Europäisches Patentamt

European Patent Office

Office européen des brevets

(11) Numéro de publication: **0 334 452**
**A1**

## (12) DEMANDE DE BREVET EUROPEEN

(21) Numéro de dépôt: **89200723.8**

(22) Date de dépôt: **22.03.89**

(51) Int. Cl.⁴: **A61M 31/00 , A61M 35/00**

(30) Priorité: **24.03.88 IT 6726088**

(43) Date de publication de la demandè:
**27.09.89 Bulletin 89/39**

(84) Etats contractants désignés:
**CH DE ES FR GB LI**

(71) Demandeur: **Ottavio, Claudio, Dr.**
**Via Ponte Romano n. 86**
**I-11027 Saint-Vincent (AO)(IT)**

(72) Inventeur: **Ottavio, Claudio, Dr.**
**Via Ponte Romano n. 86**
**I-11027 Saint-Vincent (AO)(IT)**

(54) **Instrument pour appliquer substances fluides surtout médicamenteuses.**

(57) Un instrument à usage manuel pour l'administration et l'application de substances fluides, surtout médicamenteuses, en lui renfermées, sur des surfaces ou en cavités de differentes sortes, constitué d'un récipient (2) scellable après son remplissage, pourvu d'un prolongement creux (8) lequel se joint d'une façon adhérente à l'extrémité pointue et percée (7) d'un tuyau distributeur (5), lequel, grâce à la présence sur lui d'une flasque d'appui (10), peut, à l'aide d'une simple manualité de l'opérateur, pénétrer à l'interieur du récipient (2) en le perçant. Le tuyau distributeur (5) peut servir aussi comme applicateur, parce que sur l'autre extrémité (15), on peut mettre des fibres d'ouate de coton, ou un autre materiàu approprié, avec lequel on peut étendre le fluide sur la partie qu'on doit traiter.

Fig.G

## INSTRUMENT POUR APPLIQUER SUBSTANCES FLUIDES SURTOUT MEDICAMENTEUSES

Le sujet de cette invention c'est un instrument caractérisé par un récipient qui peut être vidé par aplatissement et qui se joint avec un tuyau distributeur,le tout est affecté à l'application ou à l'administration des substances fluides surtout celles médicamenteuses,sur les tissus ou à l'interieur des cavités corporelles,ou bien d'autres fluides sur des surfaces et dans des cavités d'autre nature.

L'application des substances fluides,surtout celles médicamenteuses,est réalisé d'habitude avec l'emploi de tampons d'ouate de coton ou autre materiel,ou encore avec l'emploi de pinceaux ou d'autres instruments,lesquels ont été précédemment imbibés ou trempés dans le fluide qu'on doit appliquer; tandis que quand il s'agit de substances pâteuses,en général on prend et on applique la quantité utile directement en se servant seulment des doigts.Differentement,quand on doit introduire ou administrer une certaine quantité de fluides médicamenteux dans les cavités corporelles de l'homme ou des animaux,on emploie normalement:de petites cuillères,de petits verres, de seringues ou d'autres outils,lesquels sont remplis au moment de l'utilisation et necessittent, pour administrer leur contenu,plusieurs et compliqués mouvements des mains qui font devenir pas commode leur usage,surtout quand on travaille dans des situations difficiles. Ces désavantages ils s'ajoutent à d'autres,par exemple:les substances médicamenteuses contenues dans des récipients multidose,qui ne sont pas hermetiques,se conservent très peu dans le temps: risque de contagion pour le personnel sanitaire quand doit soigner un patient atteint d'une maladie trasmissible par simple contact;difficulté de mettre de volumineux paquets d'ouate de coton stérilisé dans les boîtes à pansement.

Le but de cette invention est de rassembler dans un seul instrument des composants particuliers,qui sont en mesure d'offrir les plus nombreux avantages possibles, donc ses caractéristiques principales sont:
- d'être un récipient destiné à contenir des substances fluides,aplatissable,cachetable,de préférence unidose,maniable;
- d'être un distributeur qui peut fonctionner aussi comme outil applicateur,qui peut avoir à son extrémité de l'ouate de coton ou bien un autre materiel qui peut s'imbiber et apte à étendre le fluide sur la surface qu'on doit traiter.

Les parties de cet instrument,qui peuvent être separées les unes des autres,sont assemblées pour former un unique instrument qui est conditionné déja prêt pour être employé,donc il présente les suivants avantages:

1) pouvoir avec des simples mouvements d'une seule main et sans devoir employer des autres instruments,exécuter les suivantes opérations:
-déconditionner l'extrémité du distributeur
- ouverture et vidage du récipient
-debit et ou application à volonté d'une exacte quantité de fluide;

2)être un instrument de petite taille,donc on le peut très facilement arrimer;

3) le récipient,scellable,peut être unidose,donc est capable de garantir une conservation assez durable à la substance qui est contenue;

4) être un distributeur qui évite à l'operateur d'avoir un contact direct avec le fluide et avec la surface qu'on doit traiter;

5)peut être employé une seule fois et conditionné d'une façon stérile;

6)d'être facilement et économiquement construible;
en effet le récipient peut avoir un prolongement creux qui se branche d'une façon adhérente à l'extrémité percée et de préférence pointue,du tuyau distributeur,qui grâce à la présence sur lui d'une flasque qui sert comme point d'appui, peut à l'aide d'une simple opération manuelle,rentrer à l'interieur du récipient en le perçant.

Par ce gendre d'instrument,qui peut être construit en tail les differentes et avec plusieurs types de materiaux,grâce à ses caractéristiques spéciales,on peut nettoyer et panser des blessures ou des écorchures,désinfecter ou traiter par des irrigations les parties anatomiques placées dans les cavités corporelles,appliquer un désinfectant topique avant d'effectuer une injection,distribuer avec un massage leger des substances thérapeutiques à usage dermatologique,distribuer des lavages à usage obstétrique-gynécologique,appliquer des substances cosmétiques,distribuer et appliquer substances collantes,et aussi réaliser toutes autres applications et débits similaires.

Quelques modes de réalisation de cette invention,sont décrites plus en détail ci-dessous ,en se référant à leur représentation schématique,que,dans les dessins annexes,est illustrée comme un simple exemple non restrictif.

DESCRIPTION DES DESSSINS

Fig. A - C'est un'illustration,en section axiale,du tuyau distributeur pourvu des fibres d'ouate de coton enroulées autour de son extrémité de débit;

Fig. A bis - en est une vue en plan d'une section faite à la hauteur de la flèche III,Fig. A ;

Fig. B - montre en section axiale le récipient pourvu du prolongement;

Fig. C - c'est un'illustration en vue laterale du bouchon pour sceller le récipient;

Fig. D - en est une vue en plan;

Fig. E - montre la vue en plan d'une section du récipient faite au niveau de la flèche IV de la Fig. B ;

Fig. F - montre en vue latérale l'instrument complet,pourvu de récipient et du tuyau distributeur,dans le quel on voit en section l'extrémité du tuyau distributeur qui est rentrée à l'interieur du récipient;

Fig. G - c'est un'illustration en vue latérale de l'instrument conditionné à l'interieur de son enveloppe scellée, pourvu du récipient,du tuyau distributeur,de la flasque mobile,dans cette illustration on voit en trasparence le récipient qui se joint,au moyen de son prolongement,avec le tuyau distributreur, avant d'être percé;

Fig. H - c'est une vue en plan de la flasque mobile;

Fig. I - c'est une vue en perspective d'un type de tuyau distributeur,porvu d'une douille de support et (en haut) du récipient correspondant;

Fig. L - montre en vue latérale la section axiale d'un type de récipient apte pour contenir une plus grande quantité de fluide,pourvu d'un bouchon et inséré à l'extrémité de son tuyau distributeur.

L'instrument d'après cette invention,comprend un récipient creux 2 qui peut avoir une forme,une taille et une capacité differentes selon l'usage auquel vient affecté;les figures B,F,G,I,montrent quelques formes de récipient avec une capacité limitée,tandis que la fig.L montre un modèle qui a une capacité plus grande;il est construit avec un materiau de préférence synthétique et sa consistence est de préférence: - plus raide dans ses parties 3 (voir figs.B,E,L) afin de permettre son branchement sur le tuyau distributeur 5 sans subir une déformation remarquable-moins raide dans ses parties 4 (voir figs.E,F,L) pour être souple et donc facilement vidable au moyen d'une pression manuelle ici exercée, - en outre sa partie 6 (voir figs. B,F,L) est construite d'une façon qu'elle soit facilement percée ou renversée par la extrémité 7 du tuyau distributeur 5,quand ceci au moment du branchement entre dans son intérieur.Le récipient 2 pour être scellable après son remplissage,peut être pourvu d'un bouchon 9 (voir figs.D,C) qui le ferme d'une façon hermétique (voir figs.B,F,L) ou bien peut être scellé d'une façon différente à condition qu'on puisse réaliser le même avantage.

L'instrument selon cette invention, pour réaliser l'avantage d'être prêt à l'usage, normalement est conditionné avec le récipient 2 déjà rempli d'une certaine quantité de fluide et, avec son prolongement 8,déjà inséré sur le tuyau distributeur 5 (voir fig.G).Au moment de s'en servir,grâce à deux forces opposées exercées par l'operateur:l'une sur le récipient 2 selon la flèche V,et l'autre sur la flasque 10 selon la flèche VI,réalise, au cas où la flasque 10 peut glisser le long de la partie 14 du tuyau 5 , - l'avantage de pouvoir déconditionner l'extrémité apte au débit 15 du susdit tuyau,en utilisant une seule main et sans se servir d'autres instruments;en suite - le branchement de l'extrémité 7 du tuyau 5 à l'interieur du récipient 2,après en avoir percé où renversé la partie 6 (voir figs.G,F)

Pendant le magasinage de l'instrument conditonné selon la figure G,pour éviter un percement accidentel du récipient 2 fait par le tuyau 5,le prolongement creux 8 est adhérent autour du tuyau 5,d'une façon élastique et peut avoir une bague circulaire 11 saillante à l'intérieur de sa cavité, d'une façon qu'il ne puisse glisser facilement vers la partie 6 du récipient 2 (voir figs.B,G,L);et, en même temps au moment du branchement,grâce à son élasticité,le prolongement 8 peut se dilater de manière que la bague 11 puisse glisser le long du tuyau distributeur 5 pour s'insérer dans la rainure périmetrale 12 qui a été ici faite (voir figs.A,F), se réalise ainsi une connexion stable et une étanchéité entre le récipient 2 et le tuyau distributeur 5 .

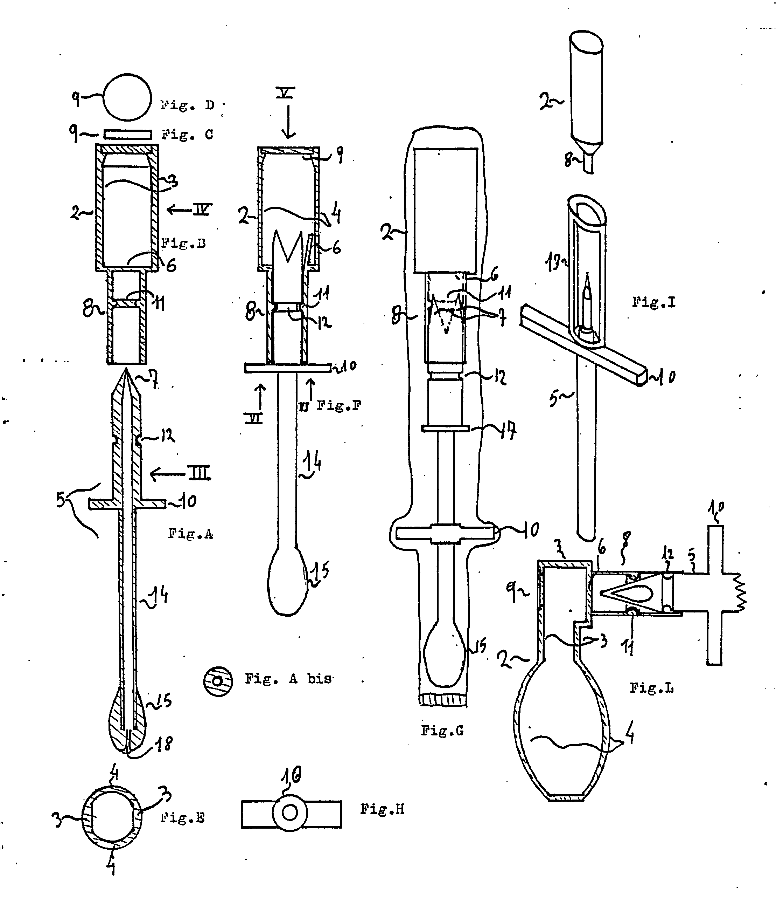

Pour empêcher que pendant la phase de branchement le récipient 2 soit déplacé de son axe d'une façon excessive,par rapport au tuyau 5,le récipient a une consistence appropriée au niveau de son prolongement 8,la même que celle de ses parties 3 (voir figs.B,E,L),tandis que le tuyau distributeur 5 il est pourvu de préférence,au niveau de son extrémité 7, d'une ou plusieurs pointes pour augmenter la surface de connexion entre les deux composants;pour obtenir le même but on peut utiliser des autres solutions,une par exemple est illustrée en perspective dans la figure 1,dans la quelle le tuyau 5 est pourvu d'une douille 13 qui réalise un support latéral et sert comme glissière pour le récipient 2 .

A la fin de la phase de branchement,le récipient 2 s'arrête, au moyen de son prolongement 8,au niveau de la flasque 10 dans le cas où celle-ci est fixe(voir fig.F),ou bien au niveau de la zone d'arrêt 17 en manchon, saillante à l'extérieur ( voir fig.G) du tuyau 5; en suite l'opérateur à l'aide d'une simple pression exercée par ses doigts sur les parois souples 4 du récipient 2,réalise le débit du fluide qui y est renfermé,à travers le tuyau 5,lequel peut être flexible au niveau de sa partie 14 et,quand il sert aussi pour appliquer,il est pourvu de préférence,au niveau de son extrémité 15,de fibres d'ouate de coton enroulées;lesquelles,quand le fluide qu'on doit appliquer est plutôt visqueux ou pâteux, sont placées d'une façon telle qu'il y, a dans leur intérieur un petit canal d'écoulement 18 (voir figs.A,F,G);au lieu de ces fibres on peut placer un pinceau ou un autre matériau qui puisse se mouiller du fluide distribué et servir aussi pour son application.

Dans les modes de réalisation illustrés et décrits,le tuyau distributeur 5,creux et percé au niveau de ses extrémités 7 et 15,est construit en une pièce,mais peut être réalisé aussi en deux ou plusieurs pièces,qui sont assemblées lors de l'usage,en outre le branchement entre le tuyau distributeur et le récipient peut être réalisé aussi par vissage ou par douille à baionette ou d'autres façons.

En outre,la flasque d'appui (10 quand est mobile,c'est-à-dire glisse le long du tuyau distributeur 5 entre l'extrémité de débit 15 et le secteur d'arrêt 17,au lieu d'être une partie de l'instrument,elle est un élément raide qui a la même fonction,mais il est une partie de l'enveloppe.

Quand l'instrument selon cette invention doit distribuer une très pétite quantité de fluide qu'il contient, les mesures de la lumière du tuyau distributeur 5,les mesures,la forme de l'extrémité 7, sont telles que déjà au moment du branchement du tuyau distributeur 5 à l'interieur du récipient 2, le fluide ici renfermé, peut arriver à l'extrémité de débit et d'application 15,sans pour ça devoir appuyer sur les parois 4 du récipient 2.

Evidemment l'instrument selon cette invention peut être construit en differents formats,en fonction de la quantité de produit qu'il doit contenir et distribuer,et en même temps, son tuyau 5 peut avoir différents diamètres et longueur et formes,selon les façons de distribution et d'application prévues.

Tous les composants peuvent être modifiés non seulment comme indiqué, mais aussi selon les différentes exigences de distribution et de fabrication,et chaque partie peut être remplacée par d'autres équivalents techniques.


**Revendications**

1 - Instrument à usage manuel pour la distribution de substances fluides en lui renfermées,sur des surfaces ou en cavités de différentes sortes,constitué d'un récipient(2) scellable après son remplissage,joint d'une façon étanche,à l'extrémité percée(7)d'un tuyau distributeur(5),lequel grâce à la présence sur lui d'une flasque d'appui(10),peut,à l'aide d'une simple manualité de l'opérateur,pénétrer à l'interieur du récipient(2) en le perçant.

2 - Instrument selon la revendication 1,caractérisé en ce que le récipient(2) est construit avec un materiau,une forme, et une consistance tels à permettre son branchement sur le tuyau distributeur(5),pour être facilement percé,sans pour ça,subir des déformations remarquables et des déplacements latéraux excessifs,et tels à permettre son vidage par une simple pression manuelle exercée sur ses parois (4).

3 - Instrument selon la revendication 1,caractérisé en ce que l'extrémité (7) du tuyau distributeur (5) qui pénètre à l'interieur du récipient (2),est pourvue d'une ou plusieurs pointes.

4 - Instrument selon la revendication 1,caractérisé en ce que le récipient (2) est démontable du tuyau distributeur(5)

5 - Instrument selon la revendication 1,caractérisé du fait que le prolongement (8) du récipient (2) adhère d'une façon élastique à l'extrémité (7) du tuyau distributeur (5) et il est pourvu d'une bague circulaire (11) qui fait saillie à l'interieur de sa cavité; elle a deux buts: a) d'éviter que pendant le magasinage, le tuyau distributeur (5) puisse percer accidentallement le récipient (2) - b) d'avoir la fonction de dispositif d'étanchéité après que le tuyau distributeur (5) est rentré à l'interieur du récipient (2), par-ce qu'elle va se placer dans la rainure périmétrale (12) faite autour du tuyau distributeur (5).

6 - Instrument selon la revendication 1,caractérisé en ce que son conditionnement est disposé d'une façon qui permet la sortie de l'extrémité de débit (15) du tuyau distributeur (5),comme consèquence de deux forces opposées exercées respectivement sur la récipient (2) et sur la flasque d'appui (10),la quelle en ce cas est mobile,soit : glisse le long du tuyau distributeur (5) entre l'extrémité de débit (15) et le secteur d'arrêt (17).

7 - Instrument selon les revendication 1 et 6,caractérisé en ce que la dite flasque d'appui (10) mobile,au lieu d'être une partie de l'instrument,elle est un élément raide qui a la même fonction,mais il est une partie de l'enveloppe.

8 - Instrument selon la revendication 1, caractérisé en ce que l'extrémité de débit (15) du tuyau distributeur(5) est disposée pour être pourvue de fibres d'ouate de coton enrou lées sur elle, ou d'un autre dispositif ou d'un autre matériau,qui s'imbibe du fluide distribué,ou quand même apte à appliquer ce fluide sur la surface qu'on doit traiter.

9 - Instrument selon les revendications 1 et 8,caractérisé en ce que le-dit dispositif de débit (15) apte à l'application est porvu dans son interieur d'un ou plusieurs canaux de débit (18) aptes à lécoulement des fluides pâteux ou très épais.

10 - Instrument selon la revendication 1,caractérisé en ce qu'il est disposé pour être commercialisé en conditionnement stérilisé, rempli du fluide qu'on doit appliquer.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-3 354 883 (J.E. SOUTHERLAND)<br>* Revendications 1-7; figures 1-3 * | 1,2,4,5 | A 61 M 31/00<br>A 61 M 35/00 |
| Y | | 3 | |
| Y | | 8-9 | |
| | --- | | |
| Y | FR-A-1 414 863 (LEMOINE)<br>* Figures 1,3,5 * | 3 | |
| | --- | | |
| Y | US-A-2 490 168 (STRAUSS)<br>* Colonne 1, lignes 39-50; colonne 2, lignes 25-45; figures 1-4 * | 8-9 | |
| | --- | | |
| X | US-A-4 201 406 (T.M. DENNEHEY et al.)<br>* Résumé; figures 1-3 * | 1-2,4, 10 | |
| | ----- | | |

DOMAINES TECHNIQUES RECHERCHES (Int. Cl.4)

A 61 M

Le présent rapport a été établi pour toutes les revendications

| Lieu de la recherche | Date d'achèvement de la recherche | Examinateur |
|---|---|---|
| LA HAYE | 29-06-1989 | MIR Y GUILLEN V. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)